# EUROPEAN PATENT APPLICATION

(11) **EP 4 506 444 A1**
(43) Date of publication of application: **12.02.2025**
(21) Application number: 23780753.2
(22) Date of filing: 29.03.2023
(51) Int. Cl.: C12N 5/071, C12Q 1/02, G01N 33/53

(54) **CONJUNCTIVAL EPITHELIUM MARKER AND USE FOR SAME**

(30) Priority: 01.04.2022 JP 2022062077
(71) Applicant: OSAKA UNIVERSITY, Suita-shi Osaka 565-0871 (JP)
(72) Inventor: NISHIDA Kohji, Suita-shi, Osaka 565-0871 (JP); HAYASHI Ryuhei, Suita-shi, Osaka 565-0871 (JP); KITAO Masahiro, Suita-shi, Osaka 565-0871 (JP)
(74) Representative: Kraus & Lederer PartGmbB
(86) International application number: PCT/JP2023/012952
(87) International publication number: WO 2023/190745

(57) **Abstract**

Disclosed herein is a method for producing a conjunctival epithelial cell population, comprising (1) differentiating pluripotent stem cells into conjunctival epithelial stem and progenitor cells, (2) collecting cells expressing a conjunctival epithelial marker from the conjunctival epithelial stem and progenitor cells, and (3) culturing the cells expressing the conjunctival epithelial marker to allow the cells to mature, wherein the conjunctival epithelial marker is selected from the group consisting of BST2, SLC2A3, AGR2, TMEM54, OLR1, TRIM29, and CITED2. Also disclosed is a method for detecting conjunctival epithelial cells, comprising detecting, from an ocular surface epithelial cell population, cells expressing a conjunctival epithelial marker selected from the group consisting of BST2, SLC2A3, AGR2, TMEM54, OLR1, TRIM29, and CITED2. Further disclosed is a method for increasing the purity of conjunctival epithelial cells in an ocular surface epithelial cell population, comprising collecting cells expressing a conjunctival epithelial marker selected from the group consisting of BST2, SLC2A3, AGR2, TMEM54, OLR1, TRIM29, and CITED2. The present invention is useful for production of a conjunctival epithelial cell population, detection of conjunctival epithelial cells, and increase in the purity of conjunctival epithelial cells.

## Description

### TECHNICAL FIELD

The present invention relates to a conjunctival epithelial marker and use thereof. In particular, the present invention relates to a conjunctival epithelial marker for detecting or purifying conjunctival epithelial cells, and methods for producing a conjunctival epithelial cell population and for increasing the purity of conjunctival epithelial cells using the conjunctival epithelial marker.

### BACKGROUND ART

Ocular surface is covered with the conjunctival epithelium and the corneal epithelium. Both epithelia play a crucial role for homeostasis of the ocular surface. Studies on human corneal epithelium have relatively progressed, revealing that corneal epithelial stem and progenitor cells reside in the corneal limbus and contribute to the maintenance of the corneal epithelium. In contrast, many details of human conjunctival epithelium remains unclear. Conjunctival epithelial stem and progenitor cells have been reported to be abundant in the conjunctival fornix, but their localization within the eye and their molecular mechanism are largely unknown (Non Patent Literature 1).

Several markers for corneal epithelial stem and progenitor cells have already been identified, but conjunctival epithelial markers that can be used to detect conjunctival epithelial stem and progenitor cells have not been reported yet. Discovery of conjunctival epithelial markers will contribute to clarifying the localization and molecular mechanism of conjunctival epithelial stem and progenitor cells, leading to elucidation of the pathology of conjunctival diseases and establishment of therapy for the diseases.

The inventor group developed 2D ocular organoid tissue (Self-formed Ectodermal Autonomous Multi-zone: SEAM). SEAM generated from human iPS cells can be induced to preferentially differentiate into corneal epithelium or conjunctival epithelium by adding a variety of growth factors at a certain timing, and the generated corneal and conjunctival epithelia contain respective stem and progenitor cells (Non Patent Literature 2, 3 and 4). However, ocular surface epithelial stem and progenitor cells in SEAM include not only conjunctival epithelial stem and progenitor cells but also corneal epithelial stem and progenitor cells. Therefore, there is a need for establishment of a preparation method that can produce a conjunctival epithelial cell population with high purity.

### CITATION LIST

### NON PATENT LITERATURE

Non patent literature 1: Gipson IK, Prog Retin Eye Res., 54, 49-63, 2016.
Non patent literature 2: Hayashi R, et al., Nature, 531(7594), 376-380, 2016.
Non patent literature 3: Hayashi R et al., Nat. Protoc., 12(4), 683-696, 2017.
Non patent literature 4: Nomi K et al., Cell Rep., 34(5), 108715, 2021.

### SUMMARY OF INVENTION

### TECHNICAL PROBLEM

An object of the present invention is to discover a conjunctival epithelial marker that can be used for detection and purification of conjunctival epithelial cells, and to provide a method for producing a conjunctival epithelial cell population using the marker, a method for detecting conjunctival epithelial cells using the marker, and a method for increasing the purity of conjunctival epithelial cells using the marker.

### SOLUTION TO PROBLEM

The present invention was made to solve the above problems and includes the following.
[1] A method for producing a conjunctival epithelial cell population, comprising
   (1) differentiating pluripotent stem cells into conjunctival epithelial stem and progenitor cells,
   (2) collecting cells expressing a conjunctival epithelial marker from the conjunctival epithelial stem and progenitor cells, and
   (3) culturing the cells expressing the conjunctival epithelial marker to allow the cells to mature,
   wherein the conjunctival epithelial marker is selected from the group consisting of BST2, SLC2A3, AGR2, TMEM54, OLR1, TRIM29, and CITED2.
[2] The production method according to the above [1], wherein the conjunctival epithelial marker is selected from the group consisting of BST2, SLC2A3, AGR2, and TMEM54.
[3] The production method according to the above [1], wherein the step (1) further comprises culturing the cells in a medium containing an EGF (Epidermal Growth Factor) receptor agonist.
[4] The production method according to the above [1], wherein the step (3) further comprises culturing the cells in a medium containing a KGF (Keratinocyte Growth Factor) receptor agonist.
[5] The production method according to the above [1], wherein the step (1) further comprises inducing an SEAM (Self-formed Ectodermal Autonomous Multi-zone) from the pluripotent stem cells.
[6] The production method according to any one of the above [1] to [5], wherein the conjunctival epithelial cell population has a sheet form.
[7] The production method according to any one of the above [1] to [5], wherein the pluripotent stem cells are iPS cells.
[8] A method for detecting conjunctival epithelial cells, comprising detecting, from an ocular surface epithelial cell population, cells expressing a conjunctival epithelial marker selected from the group consisting of BST2, SLC2A3, AGR2, TMEM54, OLR1, TRIM29, and CITED2.
[9] A method for increasing the purity of conjunctival epithelial cells in an ocular surface epithelial cell population, comprising collecting cells expressing a conjunctival epithelial marker selected from the group consisting of BST2, SLC2A3, AGR2, TMEM54, OLR1, TRIM29, and CITED2.
[10] A composition for detecting or purifying conjunctival epithelial cells, comprising a substance that specifically binds to a conjunctival epithelial marker selected from the group consisting of BST2, SLC2A3, AGR2, TMEM54, OLR1, TRIM29, and CITED2.

### ADVANTAGEOUS EFFECTS OF INVENTION

The present invention provides a method for producing a conjunctival epithelial cell population, a method for detecting conjunctival epithelial cells, and a method for purifying conjunctival epithelial cells, using a conjunctival epithelial marker that can be used for detection and purification of conjunctival epithelial cells.

### BRIEF DESCRIPTION OF DRAWINGS

Fig. 1 shows the time schedule for induction of differentiation of iPS cells into corneal epithelial stem and progenitor cells or conjunctival epithelial stem and progenitor cells in Example 1.
Fig. 2 shows immunohistochemical staining of the expression of BST2 and SLC2A3 in each tissue. The first and third rows show Merge images of the fluorescent signals of BST2 and SLC2A3 with nuclear staining, respectively. The second and fourth rows show images of the fluorescent signals of BST2 and SLC2A3, respectively.
Fig. 3 shows immunohistochemical staining of the expression of AGR2 and TMEM54 in each tissue. The first and third rows show Merge images of the fluorescent signals of AGR2 and TMEM54 with nuclear staining, respectively. The second and fourth rows show images of the fluorescent signals of AGR2 and TMEM54, respectively.
Fig. 4 shows immunohistochemical staining of the expression of OLR1 and TRIM29 in each tissue. The first and third rows show Merge images of the fluorescent signals of OLR1 and TRIM29 with nuclear staining, respectively. The second and fourth rows show images of the fluorescent signals of OLR1 and TRIM29, respectively.
Fig. 5 shows immunohistochemical staining of the expression of CITED2 in each tissue. The first row shows Merge images of the fluorescent signals of CITED2 with nuclear staining. The second row shows images of the fluorescent signals of CITED2.
Fig. 6 shows a summary of the results of the detection shown in Figs. 2 to 5.
Fig. 7 shows the expression levels of BST2, PAX6, and p63 determined by RT-qPCR before and after maturation culture of CD200⁻/CD104⁺/BST2⁺ cells (indicated as BST2+ in the figure) and CD200⁻/CD104⁺/BST2⁻ cells (indicated as BST2- in the figure) collected by FACS from cells at week 12 of differentiation into conjunctival epithelium (see Fig. 1). Expression levels of (A) BST2, (B) PAX6, and (C) p63 are shown.
Fig. 8 shows the expression levels of conjunctiva markers determined by RT-qPCR before and after maturation culture of CD200⁻/CD104⁺/BST2⁺ cells (indicated as BST2+ in the figure) and CD200⁻/CD104⁺/BST2⁻ cells (indicated as BST2- in the figure) collected by FACS from cells at week 12 of differentiation into conjunctival epithelium (see Fig. 1). Expression levels of (A) MUC5AC, (B) MUC4, (C) K13, and (D) K7 are shown.
Fig. 9 shows the expression levels of cornea markers determined by RT-qPCR before and after maturation culture of CD200⁻/CD104⁺/BST2⁺ cells (indicated as BST2+ in the figure) and CD200⁻/CD104⁺/BST2⁻ cells (indicated as BST2- in the figure) collected by FACS from cells at week 12 of differentiation into conjunctival epithelium (see Fig. 1). Expression levels of (A) K12 and (B) K3 are shown.
Fig. 10 shows immunohistochemical staining of the expression of various markers observed in cell sheets produced by maturation culture of CD200⁻/CD104⁺/BST2⁺ cells of Figs. 7 to 9.
Fig. 11 shows immunohistochemical staining of the expression of various markers observed in cell sheets produced by maturation culture of CD200⁻/CD104⁺/BST2⁻ cells of Figs. 7 to 9.
Fig. 12 shows PAS staining of a cell sheet produced by maturation culture of CD200⁻/CD104⁺/BST2⁺ cells of Figs. 7 to 9.
Fig. 13 shows the results of colony assay of CD200⁻/CD104⁺/BST2⁺ cells after maturation culture (indicated as BST2+ in the figure) and CD200⁻/CD104⁺/BST2⁻ cells after maturation culture (indicated as BST2- in the figure).
Fig. 14 shows the expression levels of various genes determined by RT-qPCR after maturation culture of CD200⁻/CD104⁺/BST2⁺ cells (indicated as BST2+ in the figure) and CD200⁻/CD104⁺/SSEA-4^{low} cells (indicated as SSEA4 low in the figure) collected by FACS from cells at week 12 of differentiation into conjunctival epithelium (see Fig. 1). Expression levels of (A) MUC5AC, (B) K13, and (C) K12 are shown.
Fig. 15 shows immunohistochemical staining of the expression of various markers observed in cell sheets produced by maturation culture of the cells of Fig. 14. Staining of (A) BST2+ and (B) SSEA4 low are shown.

### DESCRIPTION OF EMBODIMENTS

### Method for producing conjunctival epithelial cell population

The present invention provides a method for producing a conjunctival epithelial cell population. The method for producing a conjunctival epithelial cell population according to the invention includes the following steps:
(1) differentiating pluripotent stem cells into conjunctival epithelial stem and progenitor cells,
(2) collecting cells expressing a conjunctival epithelial marker from the conjunctival epithelial stem and progenitor cells, and
(3) culturing the cells expressing the conjunctival epithelial marker to allow the cells to mature.

In step (1), induction of differentiation of pluripotent stem cells into conjunctival epithelial stem and progenitor cells is initiated. Examples of the pluripotent stem cells include embryonic stem (ES) cells, embryonic stem (ntES) cells from clone embryos obtained by nuclear transplantation, spermatogonial stem (GS) cells, embryonic germ (EG) cells, induced pluripotent stem (iPS) cells, pluripotent cells (Muse cells) derived from cultured fibroblasts or myeloid stem cells, etc. The pluripotent stem cells used in the invention may be iPS cells, ES cells, or ntES cells, and are preferably iPS cells.

Conjunctival epithelial stem and progenitor cells obtained by differentiation of pluripotent stem cells include cells capable of differentiating into conjunctival epithelium, and may also include conjunctival epithelial stem cells, conjunctival epithelial progenitor cells, and cells undergoing differentiation into conjunctival epithelial stem and progenitor cells.

Induction of differentiation of pluripotent stem cells into conjunctival epithelial stem and progenitor cells may be performed by any method without any particular limitation, and the differentiation method may be selected as appropriate from known methods used for induction of differentiation of pluripotent stem cells into conjunctival epithelial stem and progenitor cells. In an embodiment, induction of differentiation of pluripotent stem cells into conjunctival epithelial stem and progenitor cells may be performed by using a method for inducing differentiation of pluripotent stem cells into 2D ocular organoid tissue (Self-formed Ectodermal Autonomous Multi-zone: SEAM) as described in Non Patent Literature 2; or may be performed by using a method for culturing cells in a medium containing an EGF (Epidermal Growth Factor) receptor agonist as described in Non Patent Literature 4; or may be performed by combining these methods described in the Non Patent Literature. The EGF receptor agonist may be EGF etc.

The culture medium for use in induction of differentiation of pluripotent stem cells into conjunctival epithelial stem and progenitor cells may be any medium suitable for use in differentiation into ocular surface epithelium. This medium is also called herein ocular surface differentiation medium (ODM). Ocular surface differentiation medium may be any medium that can be used for culture of epithelial cells (serum-free medium), including a medium used for autonomous differentiation, etc.

Ocular surface differentiation medium may further contain a ROCK inhibitor, in addition to the EGF receptor agonist. The term "ROCK inhibitor" refers to a substance that inhibits Rho kinase (ROCK: Rho-associated, coiled-coil containing protein kinase). Examples of the ROCK inhibitor that can be used herein include N-(4-pyridyl)-4β-[(R)-1-aminoethyl]cyclohexane-1α-carboxamide (Y-27632), Fasudil (HA1077), (2S)-2-methyl-1-[(4-methyl-5-isoquinolinyl)sulfonyl]hexahydro-1H-1,4-diazepine (H-1152), 4β-[(1R)-1-aminoethyl]-N-(4-pyridyl)benzene-1α-carboxamide (Wf-536), N-(1H-pyrrolo[2,3-b]pyridin-4-yl)-4β-[(R)-1-aminoethyl]cyclohexane-1α-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), and N-(6-fluoro-1H-indazol-5-yl)-6-methyl-2-oxo-4-[4-(trifluoromethyl)phenyl]-3,4-dihydro-1H-pyridine-5-carboxamide (GSK429286A).

Duration of induction of differentiation in step (1) is not limited to a particular length of time, and may be a period of time by which a conjunctival epithelial marker is expressed on cells undergoing differentiation of pluripotent stem cells into conjunctival epithelial stem and progenitor cells or on differentiated cells. For example, when differentiation of pluripotent stem cells into conjunctival epithelial stem and progenitor cells is induced by the methods described in Non Patent Literature 2 and Non Patent Literature 4, the duration of induction of differentiation in step (1) may be, for example, 10 to 14 weeks. When the differentiation is induced by a different method, preliminary studies may be performed as needed to determine the duration of induction of differentiation in step (1).

In step (2), cells expressing a conjunctival epithelial marker are collected from the conjunctival epithelial stem and progenitor cells obtained in step (1). The conjunctival epithelial marker in the invention is selected from the group consisting of BST2, SLC2A3, AGR2, TMEM54, OLR1, TRIM29, and CITED2, and is preferably selected from the group consisting of BST2, SLC2A3, AGR2, and TMEM54. BST2 is bone marrow stromal cell antigen 2 (also called CD317, Tetherin, or HM1.24). SLC2A3 is solute carrier family 2 member 3 (also called GLUT3). AGR2 is anterior gradient 2 (also called AG2, AG-2, HPC8, GOB-4, HAG-2, XAG-2, PDIA17, or HEL-S-116). TMEM54 is transmembrane protein 54 (also called BCLP, CAC1, or CAC-1). OLR1 is oxidized low density lipoprotein receptor 1 (also called LOX1, LOXIN, SLOX1, CLELC8A, or SCARE1). TRIM29 is tripartite motif containing 29 (also called ATDC). CITED2 is Cbp/p300 interacting transactivator with Glu/Asp rich carboxy-terminal domain 2 (also called ASD8, MRG1, VSD2, MRG-1, or P35SRJ). After differentiation of pluripotent stem cells is induced by the method for producing a conjunctival epithelial cell population according to the invention, the proportion of cells not expressing a conjunctival epithelial marker in conjunctival epithelial stem and progenitor cells is reduced, but the proportion of cells expressing a conjunctival epithelial marker increases, resulting in a cell population with a higher proportion of conjunctival epithelium.

Cells expressing a conjunctival epithelial marker may be collected by any method using, for example, a cell sorter, affinity chromatography, etc. For example, when a cell sorter is used, a cell suspension of the conjunctival epithelial stem and progenitor cells can be prepared, and a substance that specifically binds to each of conjunctival epithelial markers can be added to the cell suspension to collect the cells expressing the conjunctival epithelial marker. For example, when affinity chromatography is used, a cell suspension of the conjunctival epithelial stem and progenitor cells may be prepared, then the cell suspension may be run through a column filled with a carrier conjugated to a substance that specifically binds to each of conjunctival epithelial markers to allow cells expressing any of conjunctival epithelial markers to bind to the substance, and the binding between the cells and the substance may be dissociated to collect the cells expressing any of conjunctival epithelial markers.

The substance that specifically binds to a conjunctival epithelial marker may be any substance that specifically binds to BST2, SLC2A3, AGR2, TMEM54, OLR1, TRIM29, or CITED2. Such a substance may be, for example, a low molecular weight compound, a peptide, an antibody, an antibody fragment, an oligosaccharide, etc. that has binding ability to BST2, SLC2A3, AGR2, TMEM54, OLR1, TRIM29, or CITED2. Preferably, the substance is an anti-BST2 antibody, an anti-SLC2A3 antibody, an anti-AGR2 antibody, an anti-TMEM54 antibody, an anti-OLR1 antibody, an anti-TRIM29 antibody, or an anti-CITED2 antibody.

Step (2) may also include collecting the cells of interest using a known cell surface marker for use in isolation and analysis of ocular surface epithelial cells from conjunctival epithelial stem and progenitor cells. Such a known cell surface marker may be, for example, a cell surface marker that is expressed on conjunctival epithelial stem and progenitor cells, which may include, for example, CD104 and ITGB4. The known cell surface marker may be a cell surface marker that is not expressed on conjunctival epithelial stem and progenitor cells, which may include, for example, CD200. The known cell surface marker may be a cell surface marker that is expressed at a low level on conjunctival epithelial stem and progenitor cells, which may include, for example, SSEA-4. Cells of interest may be collected by any method appropriately selected from known collection methods depending on the expression of the intended cell surface markers. Collection of the cells of interest may be performed simultaneously with the step (2) or before and/or after the step (2).

In step (3), the cells expressing a conjunctival epithelial marker obtained in the step (2) are allowed to mature. Maturation of the cells expressing a conjunctival epithelial marker may be performed by any method without any particular limitation, and the maturation method may be selected as appropriate from known methods used for maturation of conjunctival epithelial stem and progenitor cells into conjunctival epithelium. In an embodiment, culturing the cells in a medium containing a KGF (Keratinocyte Growth Factor) receptor agonist may be performed as described in Non Patent Literature 4. The KGF receptor agonist may be KGF etc. Preferably, the cells expressing a conjunctival epithelial marker are allowed to mature under feeder cell-free conditions to enhance the purity of a conjunctival epithelial cell population.

The culture medium for use in maturation of the cells expressing a conjunctival epithelial marker may be any medium suitable for use in maintenance culture of ocular surface epithelial cells. This medium is also called herein ocular surface epithelium maintenance medium (OEM). Ocular surface epithelium maintenance medium may be any medium that can be used for culture of epithelial cells (serum-free medium).

Ocular surface epithelium maintenance medium may further contain a serum replacement, in addition to the EGF receptor agonist or the KGF receptor agonist. Examples of the serum replacement include albumin (e.g., lipid-rich albumin), transferrin, fatty acids, collagen precursors, trace elements (e.g., zinc, selenium), B27 (registered trademark) Supplement, N2 Supplement, etc. The concentration in the medium, for example, in the case of use of B27 Supplement, is 0.01 to 10% by weight, preferably 0.5 to 4% by weight.

The ocular surface differentiation medium and/or the ocular surface epithelium maintenance medium may further contain, as appropriate, various nutrients necessary for maintenance of growth of cells and ingredients required for induction of the differentiation, in addition to the ingredients described above. Nutrients that may be contained in the medium, may be, for example, carbon sources, such as glycerol, glucose, fructose, sucrose, lactose, honey, starch, and dextrin; hydrocarbons, such as fatty acids, fats and oils, lecithin, and alcohols; nitrogen sources, such as ammonium sulfate, ammonium nitrate, ammonium chloride, urea, and sodium nitrate; inorganic salts, such as common salt, potassium salts, phosphates, magnesium salts, calcium salts, iron salts, and manganese salts (e.g., monopotassium phosphate, dipotassium phosphate, magnesium sulfate, sodium chloride, ferrous sulfate, sodium molybdate, sodium tungstate, or manganese sulfate); various vitamins; amino acids; etc. The amount of such ingredients can be adjusted according to common technical knowledge in the art.

Duration of the step (3) is not limited to a particular length of time, and may be 2 to 10 weeks. The conjunctival epithelial cell population after maturation may be in any form. Several cell populations may separately exist, or the cells may cluster together to form aggregates, or the cells may form a sheet. Preferably, the cells form a sheet.

The conjunctival epithelial cell population produced in accordance with the invention may be a human conjunctival epithelial cell population, or a non-human organism's conjunctival epithelial cell population. The non-human organisms are not limited to particular one, and may be, for example, mammals. Examples of the mammals include monkeys (e.g., cynomolgus monkeys, rhesus monkeys, etc.), chimpanzees, dogs, cats, bovines, horses, pigs, rabbits, mice, rats, etc.

### Method for detecting conjunctival epithelial cells

The present invention also provides a method for detecting conjunctival epithelial cells (referred to as the "detection method of the invention" below). The detection method of the invention includes detecting cells expressing a conjunctival epithelial marker in an ocular surface epithelial cell population. The conjunctival epithelial marker is selected from the group consisting of BST2, SLC2A3, AGR2, TMEM54, OLR1, TRIM29, and CITED2. The term "ocular surface epithelial cell population" refers to a wide concept that includes cells capable of differentiating into ocular surface epithelium, and cells that are terminally differentiated into ocular surface epithelium. The ocular surface epithelial cell population may include conjunctival epithelial stem and progenitor cells, conjunctival epithelial cells, conjunctival epithelial goblet cells, corneal epithelial stem and progenitor cells, and corneal epithelial cells. In an embodiment, the ocular surface epithelial cell population may be those obtained by induction of differentiation of pluripotent stem cells, or a surgery sample containing ocular surface epithelium, or a cell suspension prepared from a surgery sample.

Detection of cells expressing a conjunctival epithelial marker in an ocular surface epithelial cell population may be performed by any method without any particular limitation, and the detection method may be selected as appropriate from known methods. For example, cells expressing a conjunctival epithelial marker may be detected using a label present on a substance that binds to a conjunctival epithelial marker. Alternatively, a substance that binds to a conjunctival epithelial marker may be labeled with another substance to indirectly detect cells expressing a conjunctival epithelial marker. In an embodiment, detection of cells expressing a conjunctival epithelial marker in an ocular surface epithelial cell population may be performed by detecting expression of a conjunctival epithelial marker on the surface of cells, or by detecting expression of a conjunctival epithelial marker within ocular surface epithelial cells after fixation and/or permeation of the cells.

### Method for increasing the purity of conjunctival epithelial cells

The present invention also provides a method for increasing the purity of conjunctival epithelial cells (referred to as the "purity increasing method of the invention" below). The purity increasing method of the invention includes collecting cells expressing a conjunctival epithelial marker in an ocular surface epithelial cell population. The conjunctival epithelial marker is selected from the group consisting of BST2, SLC2A3, AGR2, TMEM54, OLR1, TRIM29, and CITED2. The ocular surface epithelial cell population may be cells as exemplified above as an ocular surface epithelial cell population in the previous section regarding the detection method of conjunctival epithelial cells. Collection of cells expressing a conjunctival epithelial marker can be performed in the same manner as in the step (2) of the production method of a conjunctival epithelial cell population as described above.

### Composition for detecting or purifying conjunctival epithelial cells

The present invention also provides a composition for detecting or purifying conjunctival epithelial cells, comprising a substance that specifically binds to a conjunctival epithelial marker selected from the group consisting of BST2, SLC2A3, AGR2, TMEM54, OLR1, TRIM29, and CITED2 (referred to as the "composition of the invention" below). The composition of the invention contains a substance as exemplified above as a substance that specifically binds to the conjunctival epithelial marker in the previous section regarding the method for producing a conjunctival epithelial cell population according to the invention. The composition of the invention may further contain an additional component, such as a solvent, an additive, an excipient, etc., in addition to the conjunctival epithelial marker.

The present invention also includes a kit for detecting conjunctival epithelial cells, a cell marker for detecting or purifying conjunctival epithelial cells, etc., each of which includes a substance that specifically binds to a conjunctival epithelial marker selected from the group consisting of BST2, SLC2A3, AGR2, TMEM54, OLR1, TRIM29, and CITED2.

### EXAMPLES

The present invention will be described in more detail below with reference to Examples, but the present invention is not limited thereto.

### Example 1: Search for conjunctival epithelial markers

### 1-1 Induction of differentiation into corneal epithelium or conjunctival epithelium

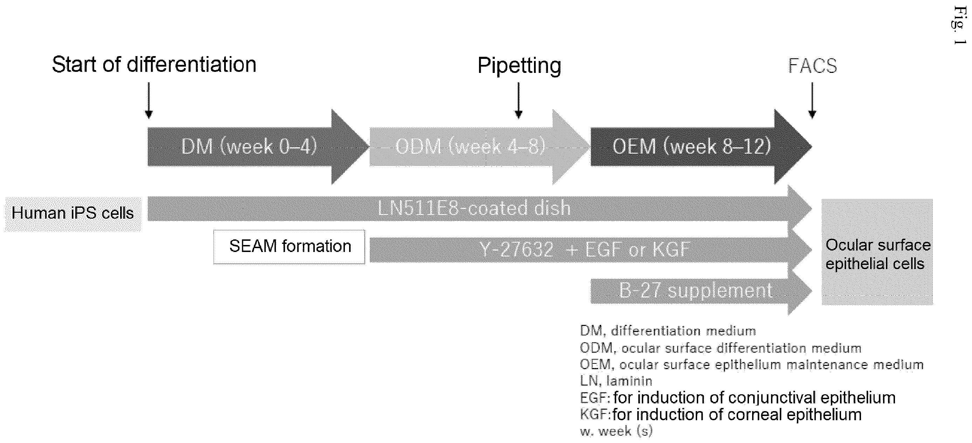

Human iPS cells were cultured in differentiation medium (DM) in culture dishes coated with laminin 511E8 fragment for 4 weeks to produce 2D ocular organoid tissue (Self-formed Ectodermal Autonomous Multi-zone: SEAM) by referring to the method described in Non Patent Literature 2. For induction of differentiation into specific ocular surface epithelial cells, the cells collected from SEAM were cultured in ocular surface differentiation medium (ODM) supplemented with Y-27632 and EGF or KGF for 4 weeks, and then the medium was replaced with ocular surface epithelium maintenance medium (OEM) supplemented with B-27 Supplement in addition to Y-27632 and EGF or KGF, and the cells were cultured for about 4 weeks, by referring to the differentiation method described in Non Patent Literature 3 and 4. EGF was used in culture for induction of differentiation into conjunctival epithelium, and KGF was used in culture for induction of differentiation into corneal epithelium. The time schedule is shown in Fig. 1.

In particular, human iPS cell line [YZWJs524 cell line (CiRA Foundation)] was seeded at a density of 100 to 700 cells/cm² in culture dishes coated with laminin 511E8, and maintained in StemFit (registered trademark) medium (Ajinomoto) for 8 to 10 days. The cells were then cultured in differentiation medium [DM; GMEM medium (Life Technologies) supplemented with 10% knockout serum replacement (KSR, Life Technologies), 1 mM sodium pyruvate (Life Technologies), 0.1 mM non-essential amino acids (Life Technologies), 2 mM L-glutamine (Life Technologies), 1% penicillin-streptomycin solution (Life Technologies) and 55 µM 2-mercaptoethanol (Life Technologies)] for 4 weeks to form SEAM. The cells in the third and fourth zones of the SEAM were collected.

For induction of differentiation into corneal epithelium, the cells collected from the third and fourth zones of the SEAM cultured in the differentiation medium were further cultured in corneal differentiation medium [1:1 (v/v) mixed medium of differentiation medium supplemented with 10 ng/mL KGF (R&D Systems) and 10 µM Y-27632 (Wako) and Cnt-20 or Cnt-PR medium (EGF/FGF2-free, CellnTEC Advanced Cell Systems)] for 4 weeks. The medium was then replaced with corneal epithelium maintenance medium [DMEM/F-12 (2:1 (v/v)) medium (Life Technologies) supplemented with 2% B-27 Supplement (Life Technologies), 1% penicillin-streptomycin solution, 10 ng/mL KGF and 10 µM Y-27632], and the cells were cultured for additional 4 weeks to induce differentiation into corneal epithelium.

For induction of differentiation into conjunctival epithelium, conjunctival differentiation medium and conjunctival epithelium maintenance medium each supplemented with 10 ng/mL EGF (Wako) instead of 10 ng/mL KGF were used.

### 1-2 Preparation of samples

The cells collected from the third and fourth zones of the SEAM at week 4 of differentiation served as undifferentiated ocular surface epithelial cell group. CD200⁻/SSEA-4^{low} (low-positive)/CD104⁺ cells and CD200⁻/SSEA-4^{high} (high-positive)/CD104⁺ cells collected by FACS at week 12 of differentiation into corneal epithelium served as cornea 1 group and cornea 2 group, respectively. CD200⁻/SSEA-4^{low}/CD104⁺ cells and CD200⁻/SSEA-4^{high}/CD104⁺ cells collected by FACS at week 12 of differentiation into conjunctival epithelium served as conjunctiva 1 group and conjunctiva 2 group, respectively. For preparation of undifferentiated ocular surface epithelial cell group, the SEAM was dissociated in Accutase at 37°C for 60 min, and the required cell populations in the third and fourth zones were obtained manually and suspended in PBS with 0.04% BSA at about 1,000 cells/µl. The cell populations of cornea 1 and 2 groups and conjunctiva 1 and 2 groups, each isolated in each fraction by FACS, were suspended in PBS with 0.04% BSA at about 1,000 cells/µl.

### 1-3 scRNAseq analysis

The five samples prepared above (undifferentiated ocular surface epithelial cell group, cornea 1 and 2 groups, and conjunctiva 1 and 2 groups) were subjected to DNA concentration measurement using Qubit (Thermo Fisher Scientific), revealing that the DNA levels of the samples were sufficiently high, 100 ng/µl or more. The libraries were prepared following the protocol of MGI Easy Universal Library Conversion Kit (App-A) (MGI). Nucleotide sequence data (FASTQ format) were obtained using the next-generation sequencer DNBSEQ-G400 (MGISEQ-200RS). The FASTQ files were then processed with the Cell Ranger "count" command using Cell Ranger pipeline ver. 3.1.0 (10x Genomics) and a reference data set [Human reference (GRCh38) dataset required for Cell Ranger]. For each sample, sufficient numbers of cells and genes were detected (data not shown), and clustering analysis was performed to group the cells based on the similarity of the gene expression pattern.

### 1-4 Analysis of ScRNAseq analytical data

Clustering analysis was performed for each sample by dimension reduction technique using t-SNE in Loupe Cell Browser (data not shown). Undifferentiated ocular surface epithelial cells were grouped into 12 clusters from 0 to 11, and cluster 1 was an undifferentiated cell population. Conjunctival epithelium was grouped into 10 clusters from 0 to 9, and clusters 2, 3, 5 and 7 were undifferentiated cell populations. Corneal epithelium was grouped into 11 clusters from 0 to 10, and clusters 0, 1 and 2 were undifferentiated cell populations. As several cluster candidates were obtained for conjunctival epithelium and corneal epithelium, Velocyto analysis was further performed (data not shown). Velocyto analysis revealed that cluster 2 of conjunctival epithelium is the most undifferentiated cell population, and cluster 0 of corneal epithelium is the most undifferentiated cell population. From the clusters identified as an undifferentiated cell population from each group, genes as potential conjunctival epithelial marker candidates were searched, and finally several conjunctival epithelial marker candidates (BST2, SLC2A3, AGR2, TMEM54, OLR1, TRIM29, and CITED2) were identified.

### 1-5 Immunohistochemical staining of human and cynomolgus monkey tissues

Bulbar conjunctiva, corneal limbus and central cornea tissues from humans and conjunctival fornix tissue from cynomolgus monkeys were embedded in O.C.T. compound (4583, Tissue-Tek) to prepare section specimens. The embedded tissue specimens were stored at -80°C, and cryosections of 5 µm were prepared using a cryostat and dried at room temperature for 30 minutes or more. The specimens were blocked with 5% NST (at room temperature for 1 hour), and then incubated with the primary antibodies shown below (at 4°C, overnight). The specimens were washed twice in TBS each for 5 minutes, and incubated with Alexa Fluor 488-conjugated secondary antibodies (1:200) and Hoechst 33342 (1:100) (at room temperature for 1 hour), and observed under a fluorescent microscope.

The following antibodies were used as the primary antibodies:
- anti-BST2 antibody (1:200; HPA017060, Atlas antibody),
- anti-SLC2A3 antibody (1:200; HPA006539, Atlas antibody),
- anti-AGR2 antibody (1:200; ab227584, abcam),
- anti-TMEM54 antibody (1:200; HPA061992, Sigma-Aldrich),
- anti-OLR1 antibody (1:200; 11837-1-AP, Proteintech),
- anti-TRIM29 antibody (1:200; sc-376125, Santa Cruz Biotechnology), and
- anti-CITED2 antibody (1:200; sc-21795, Santa Cruz Biotechnology).

The following antibodies were used as the secondary antibodies:
- Donkey anti-rabbit IgG Alexa Flour 488 conjugate (1:200; A-21206, Life Technologies), and
- Donkey anti-mouse IgG Alexa Flour 488 conjugate (1:200; A-21202, Life Technologies).

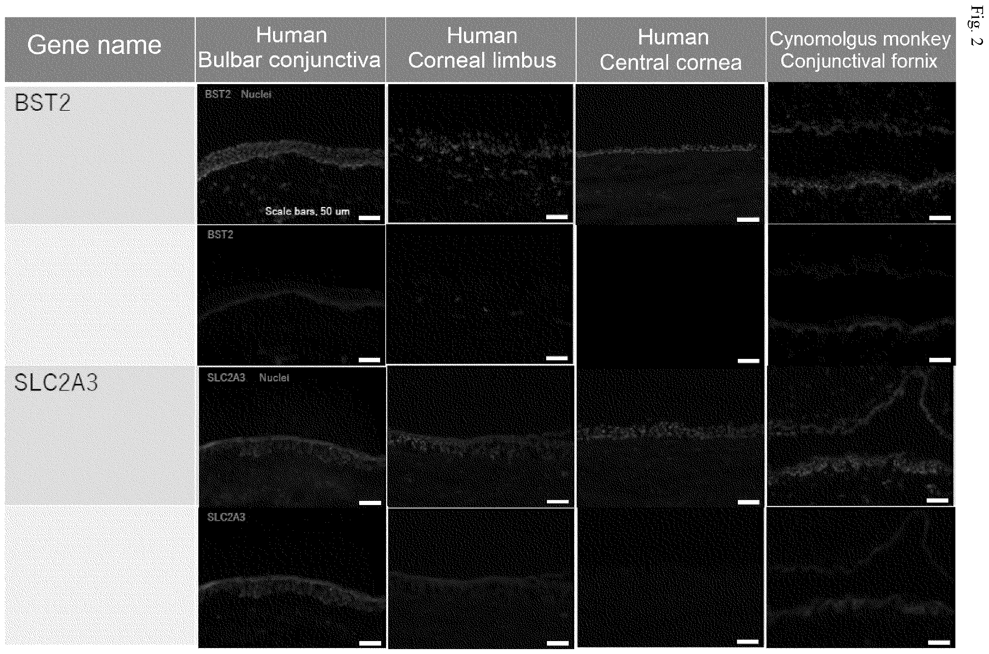

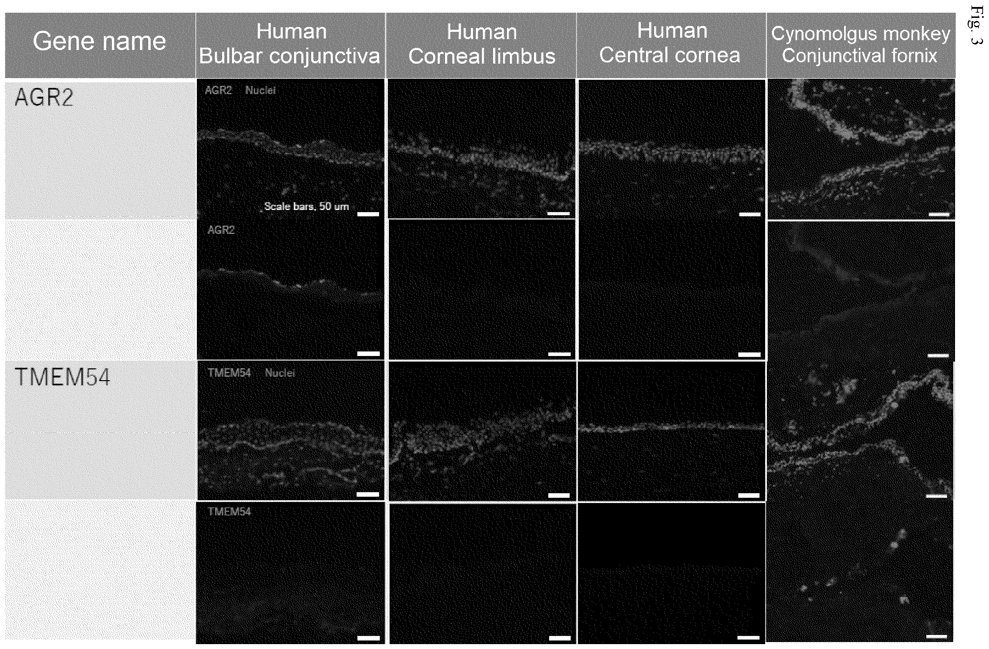

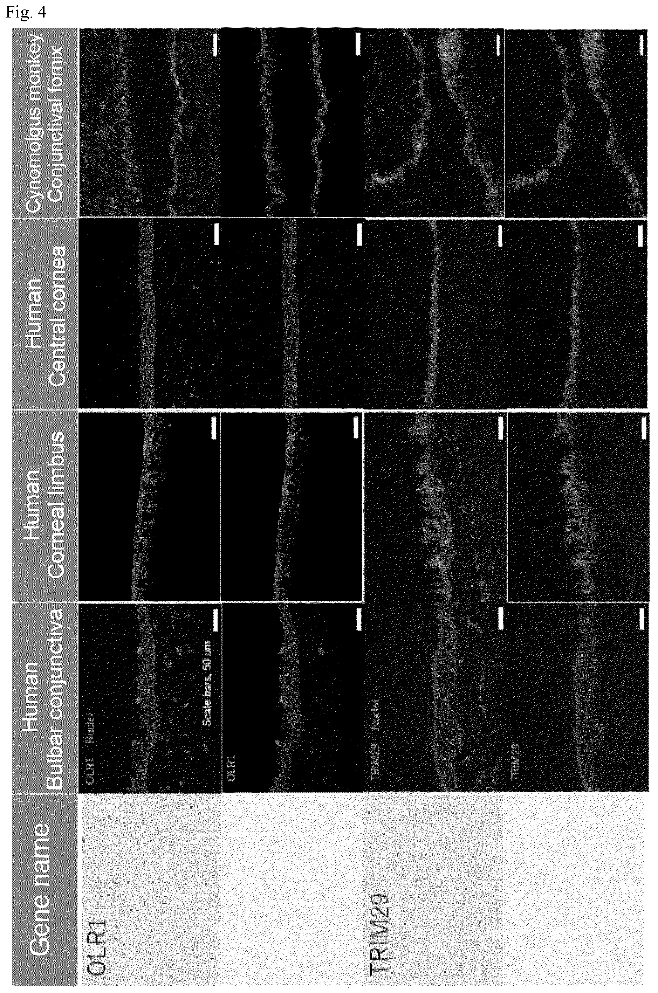

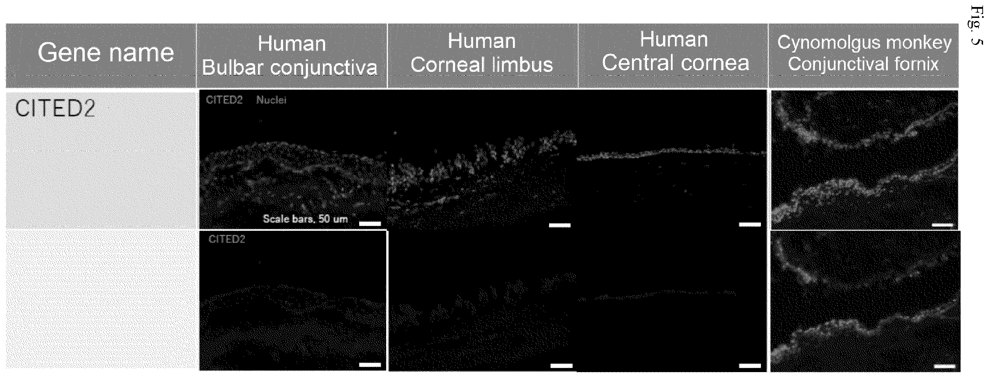

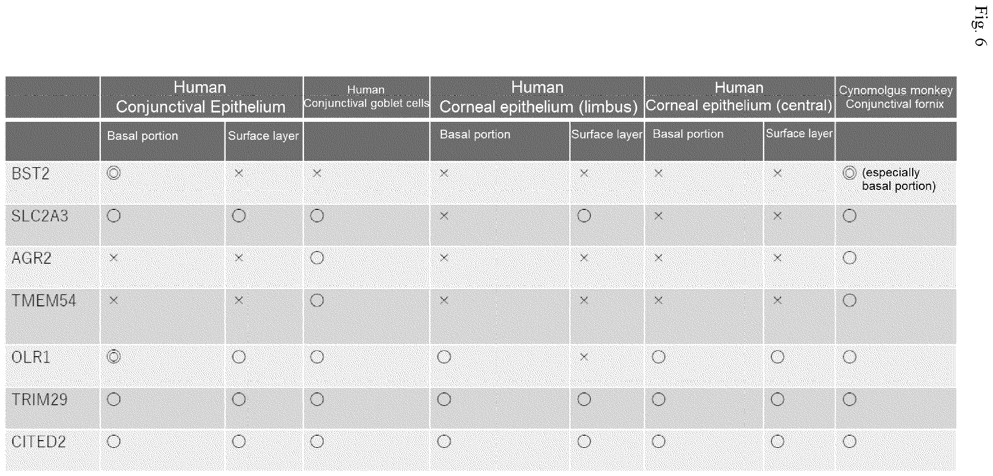

Immunohistochemical staining was performed in the tissue specimens using various types of antibodies against the conjunctival epithelial markers, and the fluorescent signals were observed, as shown in Figs. 2 to 5. The first and third rows show Merge images of the fluorescent signals of each of the conjunctival epithelial marker candidates and nuclear staining. The second and fourth rows show images of the fluorescent signals of each of the conjunctival epithelial marker candidates. The scale bar is 50 µm. The results of detection of the conjunctival epithelial markers in each tissue summarized from the results in Figs. 2 to 5 are shown in Fig. 6. In Fig. 6, "⊚" indicates that marked expression was detected, "O" indicates that expression was detected, and "X" indicates that no expression was detected. Marked expression of BST2 was detected in the basal portion of the human conjunctival epithelium and the basal portion of the conjunctival fornix of cynomolgus monkeys, but BST2 was not detected in the surface layer of the conjunctival epithelium, conjunctival goblet cells, corneal limbus or central cornea of humans. Expression of SLC2A3 was detected in the conjunctival epithelium, conjunctival goblet cells, and the surface layer of the corneal limbus of humans, as well as the conjunctival fornix of cynomolgus monkeys, but SLC2A3 was not detected in the basal portion of the corneal limbus or the central cornea of humans. Expression of AGR2 was detected in the conjunctival goblet cells of humans and the conjunctival fornix of cynomolgus monkeys, but AGR2 was not detected in the conjunctival epithelium, corneal limbus, or central cornea of humans. Expression of TMEM54 was detected in the conjunctival goblet cells of humans and the conjunctival fornix of cynomolgus monkeys, but TMEM54 was not detected in the conjunctival epithelium, corneal limbus, or central cornea of humans. Marked expression of OLR1 was detected in the basal portion of the conjunctival epithelium of humans, and expression of OLR1 was detected in the surface layer of the conjunctival epithelium, the conjunctival goblet cells, the basal portion of the corneal limbus, and the central cornea of humans, as well as the conjunctival fornix of cynomolgus monkeys, but OLR1 was not detected in the surface layer of the corneal limbus of humans. TRIM29 was detected in all of the conjunctival epithelium, conjunctival goblet cells, corneal limbus, and central cornea of humans, as well as the conjunctival fornix of cynomolgus monkeys. CITED2 was also detected in all of the conjunctival epithelium, conjunctival goblet cells, corneal limbus, and central cornea of humans, as well as the conjunctival fornix of cynomolgus monkeys. The signal sites of CITED2 correspond to the signal site of DAPI, indicating that CITED2 locates in the nucleus. All the conjunctival epithelial marker candidates (BST2, SLC2A3, AGR2, TMEM54, OLR1, TRIM29, and CITED2) were detected in the conjunctival epithelium and conjunctival goblet cells of humans, and/or the conjunctival fornix of cynomolgus monkeys, suggesting that these conjunctival epithelial marker candidates can serve as conjunctival epithelial markers.

### Example 2: Examination of efficacy as conjunctival epithelial markers

### 2-1 FACS

CD200⁻ cells were collected from the cell population at week 12 post-differentiation into the conjunctival epithelium in Example 1 by sorting using Cell Sorter SH800 (SONY). The cells were then plotted by BST2 vs CD104 to collect CD200⁻/CD104⁺/BST2⁺ cells and CD200⁻/CD104⁺/BST2⁻ cells.

The following antibodies were used for FACS:
- Mouse monoclonal anti-CD104 Alexa Fluor 647 conjugate (Cat# 624024, BD Biosciences),
- Mouse monoclonal anti-CD200 PE-Cy7 conjugate (Cat# 624052, BD Biosciences), and
- Mouse monoclonal anti-CD317 (BST2) PE conjugate (Cat# 348406, BioLegend).

### 2-2 RT-qPCR

CD200⁻/CD104⁺/ BST2⁺ cells and CD200⁻/CD104⁺/BST2⁻ cells collected by FACS were served as BST2+ and BST2- before maturation culture, respectively. CD200⁻/CD104⁺/BST2⁺ cells and CD200⁻/CD104⁺/BST2⁻ cells cultured in cell culture inserts coated with laminin 511E8 fragment (0.5 µg/cm²) for 3 to 4 weeks were served as BST2+ and BST2- after maturation culture, respectively. Medium used was the corneal epithelium maintenance medium in Example 1. Cells were treated with QIAzol, and RNA was extracted for RT-qPCR (n = 7).

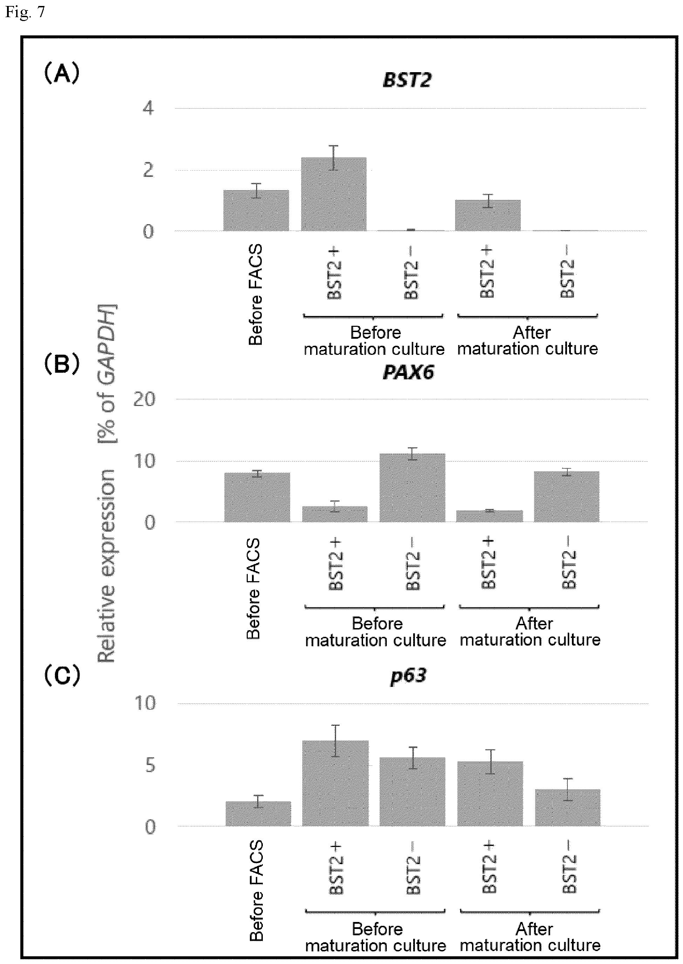

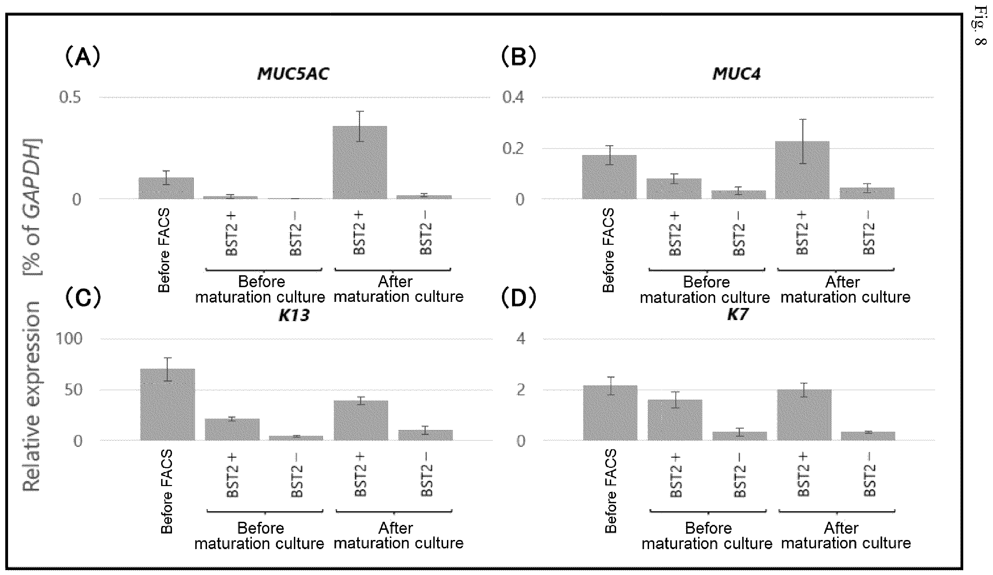

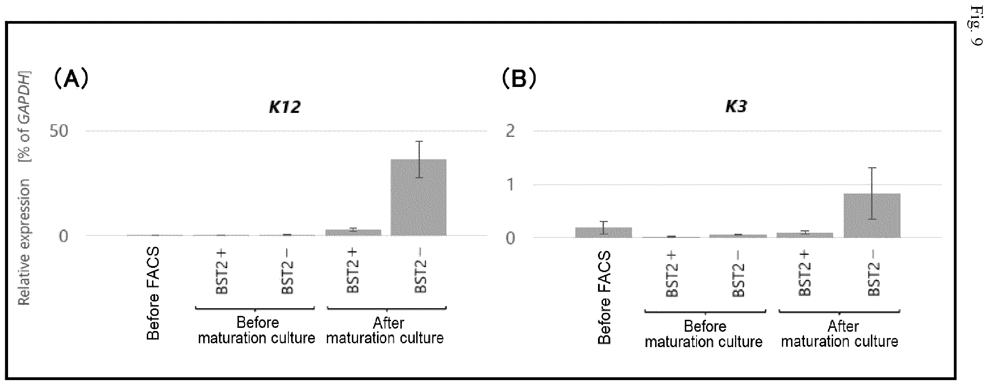

The results of RT-qPCR are shown in Figs. 7 to 9. As shown in Fig. 7, gene expression of BST2 was observed only in BST2+ before and after maturation culture, whereas gene expression of BST2 was not observed in BST2-. Gene expression of the ocular surface markers PAX6 and p63 was observed in both cells before and after maturation culture. As shown in Fig. 8, gene expression levels of MUC5AC, MUC4, K13 and K7, known as conjunctiva markers, were significantly increased in BST2+ after maturation culture. However, as shown in Fig. 9, gene expression levels of K12 and K3, known as cornea markers, were significantly increased in BST2- after maturation culture.

### 2-3 Immunohistochemical staining

CD200⁻/CD104⁺/BST2⁺ cells (referred to as BST2+ below) or CD200⁻/CD104⁺/BST2⁻cells (referred to as BST2- below) were subjected to maturation culture in the same manner as in the above section 2-2 to collect cell sheets formed by the cells. Cryosections were prepared in the same manner as in Example 1, and dried at room temperature for 30 minutes or more. The specimens were blocked with 5% NST (at room temperature for 1 hour), and then incubated with the primary antibodies shown below (at 4°C, overnight). The specimens were washed twice in TBS each for 5 minutes, and incubated with Alexa Fluor 488-conjugated secondary antibodies (1:200) and Hoechst 33342 (1:100) (at room temperature for 1 hour), and observed under a fluorescent microscope.

The following antibodies were used as the primary antibodies:
- anti-MUC5AC antibody (1:100; sc-33667, Santa Cruz Biotechnology),
- anti-K13 antibody (1:200; ab16112, abcam),
- anti-K7 antibody (1:200; ab9021, abcam),
- anti-PAX6 antibody (1:300; sc-11357, Santa Cruz Biotechnology),
- anti-BST2 antibody (1:200; HPA017060, Atlas antibody),
- anti-MUC4 antibody (1:200; sc-33654, Santa Cruz Biotechnology),
- anti-K12 antibody (1:1000; ab185627, abcam),
- anti-K3 antibody (1:200; cat#61807, PROGEN Biotechnik), and
- anti-p63 antibody (1:200; sc-8431, Santa Cruz Biotechnology).

The following antibodies were used as the secondary antibodies:
- Donkey anti-rabbit IgG Alexa Flour 488 conjugate (1:200; A-21206, Life Technologies), and
- Donkey anti-mouse IgG Alexa Flour 488 conjugate (1:200; A-21202, Life Technologies).

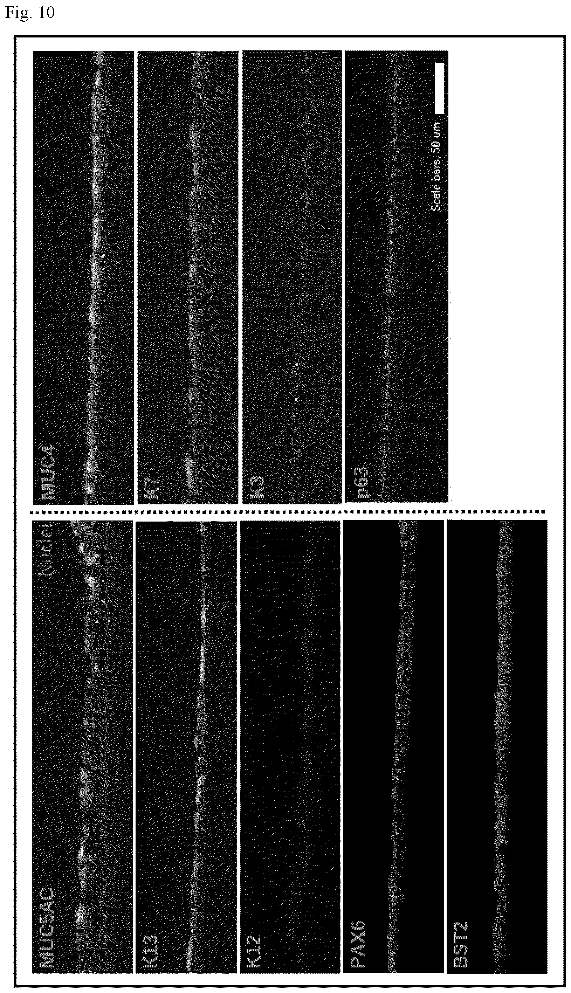

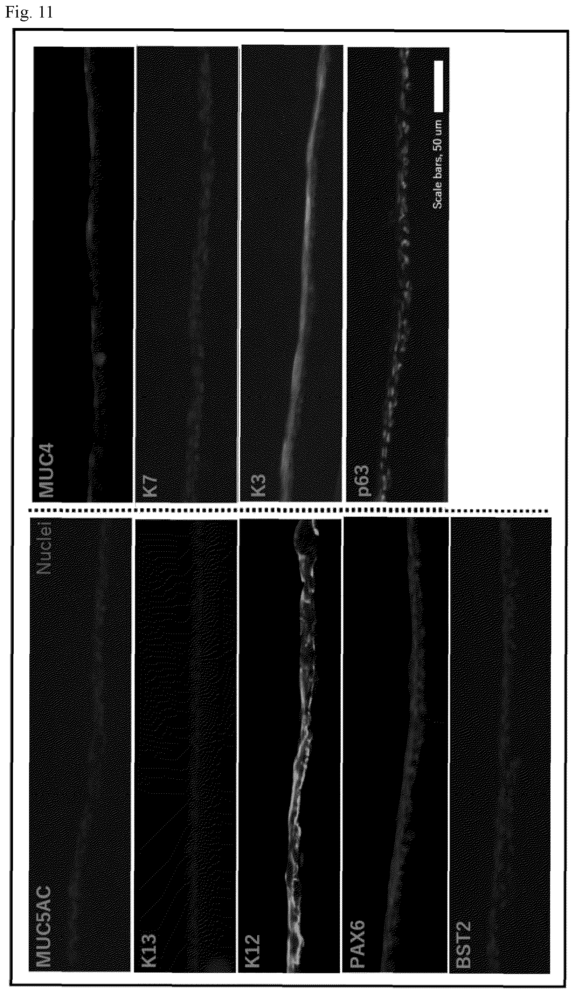

Figs. 10 and 11 show fluorescent signals observed in immunohistochemical staining in the cell sheets using various antibodies. The scale bar is 50 µm. Conjunctiva markers MUC5AC, MUC4, K13, and K7, as well as ocular surface markers PAX6 and p63 were detected in the cell sheets produced by maturation culture of BST2+, whereas cornea markers K12 and K3 were not detected in the cell sheets (Fig. 10). However, conjunctiva markers MUC5AC, MUC4, K13, and K7 were not detected in the cell sheets produced by maturation culture of BST2-, whereas cornea markers K12 and K3, as well as ocular surface markers PAX6 and p63 were detected in the cell sheets (Fig. 11). BST2 in the cell sheets shows a similar tendency to BST2 in RT-qPCR after maturation culture: BST2 was detected in the cell sheets produced by maturation culture of BST2+, whereas no BST2 was detected in the cell sheets produced maturation culture of BST2-.

### 2-4 PAS staining

The cell sheet produced by maturation culture of BST2+ was stained using a PAS staining kit (MERCK KGaA). The slide was observed with Axio Imager.A2 (Carl Zeiss).

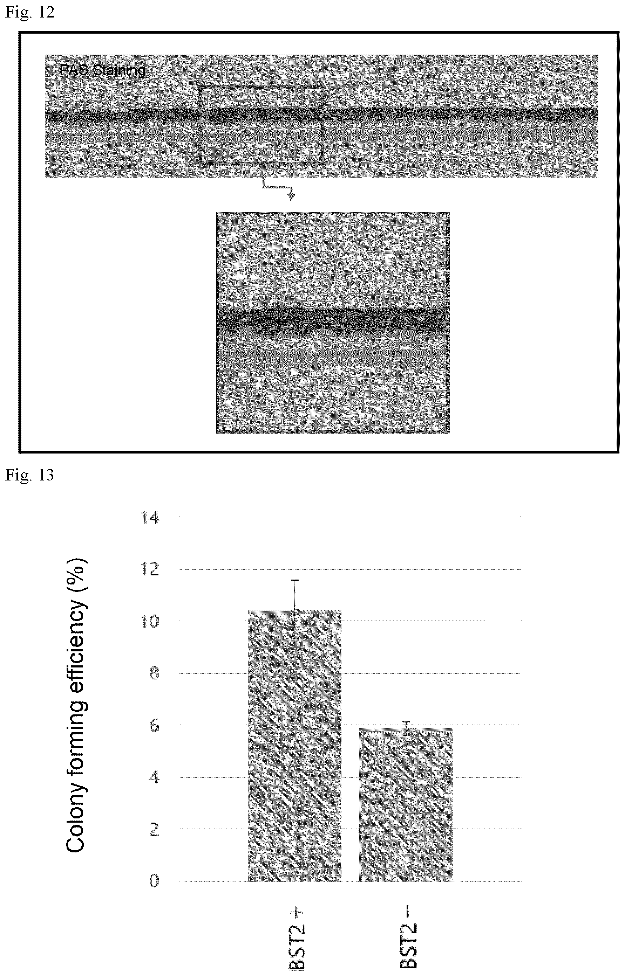

Fig. 12 shows PAS staining of the cell sheet produced by maturation culture of B ST2+. It was demonstrated that conjunctival goblet cells reside on the surface of the cell sheet.

### 2-5 Colony assay

BST2+ or BST2- collected by FACS were seeded at 2000 to 5000 cells/well on NIH-3T3 feeder cells treated with mitomycin C in 12-well plates. The cells were then cultured for 10 to 14 days at 37°C in KCM medium supplemented with 20 ng/mL KGF and 10 mM Y-27632 [3:1 (v/v) mixed medium of glutamine-free DMEM and Nutrient Mixture F-12 Ham (Thermo Fisher Scientific) supplemented with 5% FBS (Japan Bio Serum), 0.4 µg/mL hydrocortisone succinate (Wako), 2 nM 3,3',5-triiodo-L-thyronine sodium salt (MP Biomedicals), 1 nM cholera toxin (List Biological Laboratory), 2.25 µg/mL bovine transferrin HOLO Form (Thermo Fisher Scientific), 2 mM L-glutamine, and 0.5% insulin transferrin selenium solution (Thermo Fisher Scientific)]. The colonies were fixed with 10% formaldehyde neutral buffer solution at room temperature for 1 hour, and stained with rhodamine B (Wako) at room temperature for 2 hours. The number of colony formation was counted under a dissecting microscope. The number of colony formation after culture was divided by the number of the seeded cells to calculate the colony forming efficiency (CFE) (n = 5).

The results of colony assay are shown in Fig. 13. It was demonstrated that both of BST2+ and BST2- have colony forming ability. Colony forming efficiency of BST2+ was higher than that of BST2-.

### Example 3: Comparison with conventional methods

### 3-1 FACS

CD200⁻/CD104⁺/BST2⁺ cells collected in Example 2 were used. As a conventional method, CD200⁻/CD104⁺/SSEA-4^{low} cells were collected from a cell population at week 12 of differentiation into conjunctival epithelium in the same manner as in conjunctiva 1 group of Example 1. The term "low-positive" refers to an expression level that is moderately lower than expression in normal cells and that is around between positive and negative.

### 3-2 RT-qPCR

CD200⁻/CD104⁺/BST2⁺ cells and CD200⁻/CD104⁺/SSEA-4^{low} cells were cultured in the corneal epithelium maintenance medium of Example 1 in cell culture inserts coated with laminin 511E8 fragment (0.5 µg/cm²) for 3 to 4 weeks, and served as BST2+ and SSEA4 low, respectively. Cells were treated with QIAzol, and RNA was extracted for RT-qPCR. Gene expression levels of MUC5AC, K13, and K12 were assessed by RT-qPCR (n= 3).

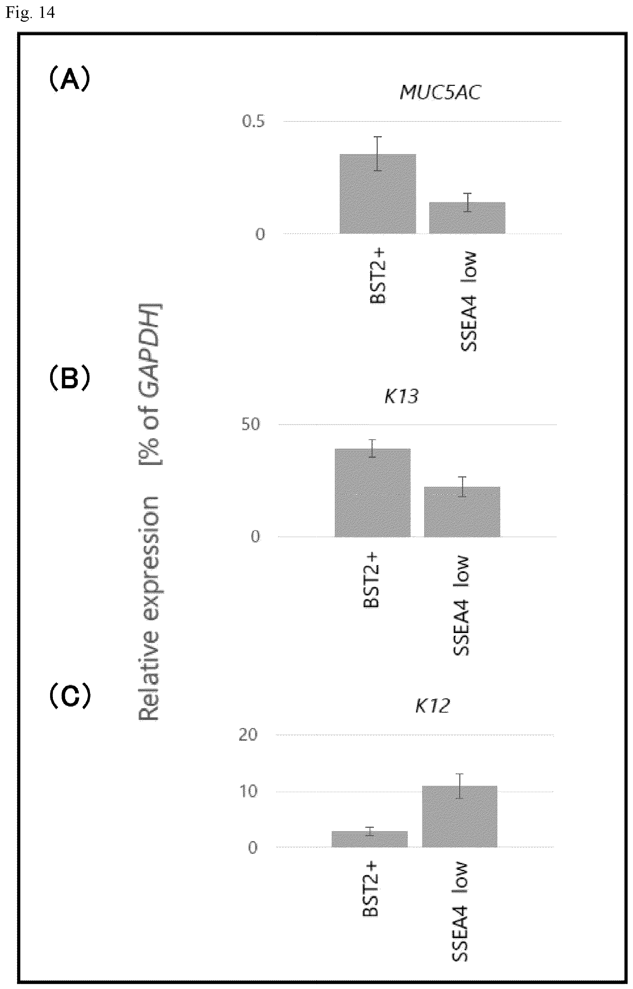

The results of RT-qPCR are shown in Fig. 14. Gene expression levels of the conjunctival goblet cell marker MUC5AC and the conjunctival epithelial cell marker K13 were higher in BST2+ than those in SSEA4 low served as a conventional method. Significant gene expression of the corneal epithelial cell marker K12 was observed in SSEA4 low served as a conventional method, but gene expression of K12 in BST2+ was very low.

### 3-3 Immunohistological staining

The results of immunohistochemical staining of MUC5AC, K13, and K12 were compared in the same as manner as in 2-3 of Example 2 except that cell sheets produced by maturation culture of BST2+ and cell sheets produced by maturation culture of SSEA4 low were used.

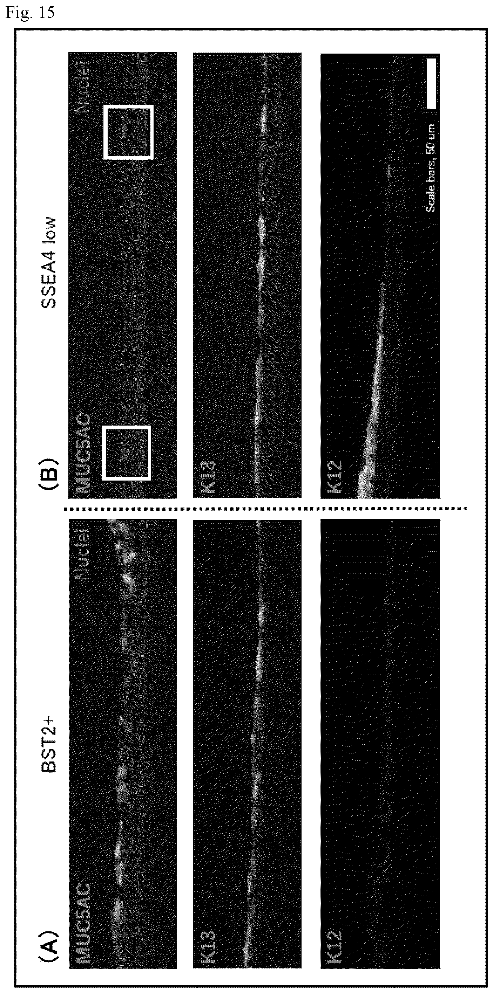

The cell sheets produced by maturation culture of BST2+ or SSEA4 low were subjected to immunohistochemical staining using various antibodies. Fig. 15 shows fluorescent signals observed in the cell sheets. (A) shows the cell sheets produced by maturation culture of BST2+, and (B) shows the cell sheets produced by maturation culture of SSEA4 low as a conventional method. The scale bar is 50 µm. Conjunctival epithelial cell marker K13 was detected in both of the cell sheets produced by maturation culture of BST2+ and the cell sheets produced by maturation culture of SSEA4 low. Conjunctival goblet cell marker MUC5AC was observed at a higher proportion in the cell sheets produced by maturation culture of BST2+ as compared with that in the cell sheets produced by maturation culture of SSEA4 low. Corneal epithelial cell marker K12 was detected in the cell sheets produced by maturation culture of SSEA4 low as a conventional method, indicating that the sheets contained cells that differentiated into corneal epithelial cells after maturation culture. In summary, CD200⁻/CD104⁺/BST2⁺ cells can be collected and used to prepare a conjunctival epithelial sheet with higher purity than that in a sheet prepared by a conventional method.

## Claims

1. A method for producing a conjunctival epithelial cell population, comprising
(1) differentiating pluripotent stem cells into conjunctival epithelial stem and progenitor cells,
(2) collecting cells expressing a conjunctival epithelial marker from the conjunctival epithelial stem and progenitor cells, and
(3) culturing the cells expressing the conjunctival epithelial marker to allow the cells to mature,
wherein the conjunctival epithelial marker is selected from the group consisting of BST2, SLC2A3, AGR2, TMEM54, OLR1, TRIM29, and CITED2.

2. The production method according to claim 1, wherein the conjunctival epithelial marker is selected from the group consisting of BST2, SLC2A3, AGR2, and TMEM54.

3. The production method according to claim 1, wherein the step (1) further comprises culturing the cells in a medium containing an EGF (Epidermal Growth Factor) receptor agonist.

4. The production method according to claim 1, wherein the step (3) further comprises culturing the cells in a medium containing a KGF (Keratinocyte Growth Factor) receptor agonist.

5. The production method according to claim 1, wherein the step (1) further comprises inducing an SEAM (Self-formed Ectodermal Autonomous Multi-zone) from the pluripotent stem cells.

6. The production method according to any one of claims 1 to 5, wherein the conjunctival epithelial cell population has a sheet form.

7. The production method according to any one of claims 1 to 5, wherein the pluripotent stem cells are iPS cells.

8. A method for detecting conjunctival epithelial cells, comprising detecting, from an ocular surface epithelial cell population, cells expressing a conjunctival epithelial marker selected from the group consisting of BST2, SLC2A3, AGR2, TMEM54, OLR1, TRIM29, and CITED2.

9. A method for increasing the purity of conjunctival epithelial cells in an ocular surface epithelial cell population, comprising collecting cells expressing a conjunctival epithelial marker selected from the group consisting of BST2, SLC2A3, AGR2, TMEM54, OLR1, TRIM29, and CITED2.

10. A composition for detecting or purifying conjunctival epithelial cells, comprising a substance that specifically binds to a conjunctival epithelial marker selected from the group consisting of BST2, SLC2A3, AGR2, TMEM54, OLR1, TRIM29, and CITED2.
